# FASCICULE DE BREVET EUROPEEN

(11) **EP 1 086 128 B1**
(45) Date de publication et mention de la délivrance du brevet: **17.09.2008**
(21) Numéro de dépôt: 99923687.0
(22) Date de dépôt: 08.06.1999
(51) Int. Cl.: C07K 14/415, C12N 15/29, C12N 15/82, C12N 15/10, A01H 5/00

(54) **PROTEINE VEGETALE A MOTIFS WD40 REPETES, ACIDE NUCLEIQUE CODANT POUR LADITE PROTEINE, ET LEURS APPLICATIONS**
PFLANZENPROTEIN MIT WD40 WIEDERHOLUNGSMUSTERN, FÜR DIESE PROTEIN KODIERENDE NUKLEINSÄURE UND VERWENDUNG DAVON
PLANT PROTEIN WITH REPEATED WD40 MOTIFS, NUCLEIC ACID CODING FOR SAID PROTEIN, AND USES THEREOF

(30) Priorité: 08.06.1998 FR 9807174
(43) Date de publication de la demande: 28.03.2001
(73) Titulaire: CENTRE NATIONAL DE LA RECHERCHE SCIENTIFIQUE (CNRS), 75794 Paris Cedex 16 (FR)
(72) Inventeur: KONDOROSI, Eva, F-91190 Gif sur Yvette (FR); CEBOLLA, Angel, F-91190 Gif sur Yvette (FR); KONDOROSI, Adam, F-91190 Gif sur Yvette (FR)
(74) Mandataire: Vialle-Presles, Marie José
(86) Numéro de dépôt international: PCT/FR1999/001342
(87) Numéro de publication internationale: WO 1999/064451

(56) Documents cités:
- WO-A-95/21917
- WO-A-98/03631
- M. LUO ET AL.,: "Cloning and characterisation of a carrot cDNA coding for a WD repeat protein homologous to Drossophila fizzy, human p55CDC and yeast CDC20 proteins" PLANT MOLECULAR BIOLOGY, vol. 34, no. 2, 1 mai 1997 (1997-05-01), pages 325-330, XP002094573 cité dans la demande
- DATABASE EMBL NUCLEOTIDE AND PROTEIN SEQUENCES, - 1 juillet 1997 (1997-07-01) XP002094591 HINXTON, GB
- MCKHANN H ET AL.,: "Cloning of a WD-repeat-containing gene from alfalfa (Medicago sativa): A role in hormone-mediated cell division?" PLANT MOLECULAR BIOLOGY, vol. 34, no. 5, 1997, pages 771-780, XP000857490
- S.J. SIGRIST AND C.F. LEHNER: "Drosophila fizzy-related down-regulates mytotic cyclins and is required for cell proliferation arrest and entry into endocycles" CELL, vol. 90, 1997, pages 671-681, XP002094574 cité dans la demande
- S. YAMAGUCHI ET AL.,: "A WD repeat protein controls the cell cycle and differentition by negatively regulating Cdc2/B-type cyclin complexes." MOLECULAR BIOLOGY OF THE CELL, vol. 8, no. 12, 1997, pages 2475-2486, XP002094575 cité dans la demande
- M. SCHWAB ET AL.,: "Yeast Hct1 is a regulator of Clb2 cyclin proteolysis" CELL, vol. 90, 1997, pages 683-693, XP002094576 cité dans la demande
- NEER E J ET AL: "The ancient regulatory-protein family of WD-repeat proteins" NATURE, vol. 371, 22 septembre 1994 (1994-09-22), pages 297-300, XP002082119 cité dans la demande
- PATTON E E ET AL: "Combinatorial control in ubiquitin-dependent proteolysis: don't Skp the F-box hypothesis" TRENDS IN GENETICS, vol. 14, no. 6, 1 juin 1998 (1998-06-01), page 236-243 XP004121083
- HILT W ET AL: "Proteasomes: destruction as a programme" TIBS TRENDS IN BIOCHEMICAL SCIENCES, vol. 21, no. 3, mars 1996 (1996-03), page 96-102 XP004050935
- BAI C ET AL: "SKP1 connects cell cycle regulators to the ubiquitin proteolysis machinery through a novel motif, the F-box" CELL, vol. 86, no. 2, 26 juillet 1996 (1996-07-26), pages 263-274, XP002082118
- DATABASE EMBL NUCLEOTIDE AND PROTEIN SEQUENCES, - 28 septembre 1998 (1998-09-28) XP002094577 HINXTON, GB
- DATABASE EMBL NUCLEOTIDE AND PROTEIN SEQUENCES,6 avril 1999 (1999-04-06), XP002125115 HINXTON, GB
- CEBOLLA A ET AL., : "The mitotic inhibitor ccs52 is required for endoreduplication and ploidy-dependent cell enlargement in plants" EMBO, vol. 18, no. 16, août 1999 (1999-08), pages 4476-4484, XP002125116

## Description

L'invention est relative au clonage de gènes intervenant dans la régulation de la division cellulaire chez les végétaux, et à leurs utilisations.

La plupart des organes végétaux se développent après la germination, par différenciation à partir des méristèmes. Préalablement à la différenciation, se produisent dans les méristèmes le ralentissement puis l'arrêt du cycle de division cellulaire. Simultanément, on observe fréquemment une augmentation de la taille des cellules, et une réplication du génome non accompagnée de mitose, dénommée « endoréplication ». L'endoréplication est un phénomène bien connu lors du développement de tissus de réserve ; KOWLES [Genome, 35, pp. 68-77, (1992)] mentionne ainsi une ploïdie de 6C à 384C lors du développement de l'endosperme chez le maïs.

Les phénomènes intervenant lors de l'arrêt de la division cellulaire précédant la différenciation jouent un rôle essentiel dans le développement et l'ontogénèse végétale. Les mécanismes impliqués dans ces phénomènes sont encore mal connus ; il semble que l'inhibition du facteur promoteur de la phase M, et l'induction des protéine-kinases de la phase S (GRAFI, Science, 269, pp. 1262-1264, (1995)] seraient impliqués. Toutefois, on n'a jusqu'à présent identifié aucun facteur directement impliqué dans ce mécanisme chez les plantes.

Les Inventeurs ont entrepris l'étude de ce mécanisme, dans le but de découvrir des moyens de le contrôler, et d'agir par son intermédiaire sur le développement et l'ontogenèse végétale.

Ils ont choisi comme modèle d'étude le système symbiotique *Rhizobium*/légumineuses. Dans ce système, les facteurs Nod de nature lipooligosaccharidique, produits par les *Rhizobium*, constituent des signaux mitogènes qui induisent localement la formation d'un nouveau méristème, à partir duquel se différencient les cellules formant les nodosités racinaires [TRUCHET, Nature, 351, pp. 670-673, (1991) ; YANG, Plant Cell, 6, pp. 1415-1426, (1994) ; SAVOURE, EMBO.J., 13, pp. 1093-1102, (1994)]. Les nodosités comprennent 3 zones principales : une zone apicale, constituée de cellules méristématiques ; une zone intermédiaire d'invasion, ou de différenciation (zone II), où intervient l'infection des cellules par les bactéries, ainsi que l'arrêt de la division cellulaire, accompagné d'endoréplication et d'augmentation de la taille des cellules, et suivi par leur différenciation ; et une zone de fixation (zone III), formée de cellules différenciées infectées par les bactéries, et où intervient la fixation de l'azote.

Au cours de cette étude, les Inventeurs ont isolé, à partir de nodosités de luzerne (*Medicago sativa*), un gène, dénommé ci-après *ccs52*, jouant un rôle essentiel dans l'arrêt du cycle cellulaire et l'induction de l'endoréplication. En utilisant une sonde d'ADNc du gène *ccs52* de *Medicago sativa* ils ont également isolé un gène homologue chez *Medicago truncatula*.

Les gènes *ccs52* de *Medicago sativa* (*ccs52Ms)*, et de *Medicago truncatula* (*ccs52Mt*) codent pour un polypeptide de 475 acides aminés, ayant une masse moléculaire théorique de 52 kDa. Ces polypeptides sont respectivement dénommés ci-après CCS52Ms et CCS52Mt ; les séquences de CCS52Ms et CCS52Mt ne diffèrent que par 2 résidus en positions 16 (R/G) et 141 (V/I).

Ces 2 protéines comprennent des motifs WD répétés, et peuvent ainsi être rattachées à la superfamille des protéines à motifs WD répétés.

Les motifs WD répétés comprennent environ 40 aminoacides contenant un certain nombre d'acides aminés conservés dont le motif WD (Trp-Asp) qui se situe fréquemment à une extrémité du motif répété [NEER et al., Nature, 371, pp. 297-300, (1994)]. Les membres de cette famille régulent différentes fonctions, telles que la transduction de signal, la transcription, l'épissage de pré-ARNm, l'organisation du cytosquelette, la fusion vésiculaire ou le cycle cellulaire. Bien que la structure générale soit globalement similaire dans toutes les protéines, la grande variété fonctionnelle des motifs WD répétés suggère que ces motifs se sont différenciés et sont devenus fonctionnellement spécialisés. Une homologie fonctionnelle se reflète dans le nombre de motifs WD répétés, par une homologie importante des motifs WD répétés à des positions équivalentes dans différentes protéines, par rapport à d'autres motifs répétés dans les mêmes protéines, et par une similarité significative des extrémités C et N terminales.

La comparaison de la séquence de CCS52Ms avec les séquences de protéines connues, en utilisant le programme GAP de GENETICS COMPUTER GROUP [paramètres : gap weight : 1,000 ; length weight : 0,100 ; average match : 0,540 ; average mismatch : 0,396] a fait apparaître une homologie élevée avec des protéines à motifs WD40 répétés qui interviennent dans la régulation du cycle cellulaire, et plus spécifiquement, avec les protéines FZR de Drosophile (57% d'identité), HCT1 de *Saccharomyces cerevisiae* (46% d'identité), et SRW1 de *Schizosaccharomyces pombe* (52% d'identité), qui appartiennent à la famille « fizzy-related » (FZR). Les recherches effectuées dans des bases de données de séquences en utilisant le programme BLAST [ALTSCHUL et al. Nucleic Acids Res. 25:3389-3402, (1997)] ont également fait apparaître une homologie importante de CCS52Ms avec les protéines FZR de Drosophile (56% d'identité ; 70% de similarité), et SRW1 de *Schizosaccharomyces pombe* (51% d'identité ; 67% de similarité) mentionnées ci-dessus, ainsi qu'avec le produit du gène *fzr* de *X. laevis* (58% d'identité ; 73% de similarité).

Les protéines FZR,induisent la dégradation des cyclines mitotiques, et interviennent dans la transition entre la prolifération et la différenciation cellulaire. Il a ainsi été montré chez la Drosophile que le gène fzr est exprimé en fin de prolifération cellulaire pendant l'embryogénèse. Le produit de ce gène entraîne une diminution des cyclines mitotiques, et est nécessaire pour l'arrêt de la prolifération cellulaire et le début des endocycles [SIGRIST et LEHNER, Cell, 90, pp. 671-681, (1997)]. Chez *Saccharomyces cerevisiae*, HCT1 est nécessaire pour la protéolyse de la cycline mitotique, Clb2 [SCHWAB et al., Cell, 90, pp. 683-693, (1997)]. Chez *Schizosaccharomyces pombe,* le produit du gène *srwl* contrôle le cycle cellulaire et la différenciation en régulant négativement les complexes Cdc2/CDC13 (cycline de type mitotique) [YAMAGUCHI et al., Mol. Biol. Cell., 8, 2475-2486, (1997)]. Les protéines FZR ont donc un rôle différent de celui des autres protéines à motifs WD répétés, qui interviennent au niveau de la prolifération cellulaire.

Chez les plantes, aucune protéine de la famille FZR n'a été décrite antérieurement à CCS52Ms.

L'existence d'un gène codant pour une protéine à motifs WD40 répétés et son isolement à partir d'ADNc de carotte ont été récemment décrits [LUO et al., Plant Mol. Biol., 34, pp. 325-330, (1997)]. Cependant, le produit de ce gène présente une plus faible homologie (44% d'identité et 63% de similarité sur la comparaison de séquences effectuée avec le programme BLAST) avec la protéine CCS52Ms, que les protéines FZR d'invertébrés et de levure ; cette protéine de carotte est apparentée aux protéines cdc20, p55, et fizzy, et appartient donc à un sous-groupe de protéines à motifs WD40 répétés distinct du sous-groupe FZR.

La recherche d'homologues de CCS52Ms dans une base de données du génome d'*Arabidopsis thaliana* a fait apparaître une séquence peptidique déduite d'un clone génomique (AB005230) et présentant 64% d'identité avec CCS52Ms, ce qui montre l'existence d'homologues du gène *ccs52Ms* chez d'autres plantes. Une autre séquence peptidique également déduite d'un clone génomique d'*Arabidopsis thaliana* (AL031018, publiée le 17 septembre 1998) présente 80% d'identité avec CCS52Ms.

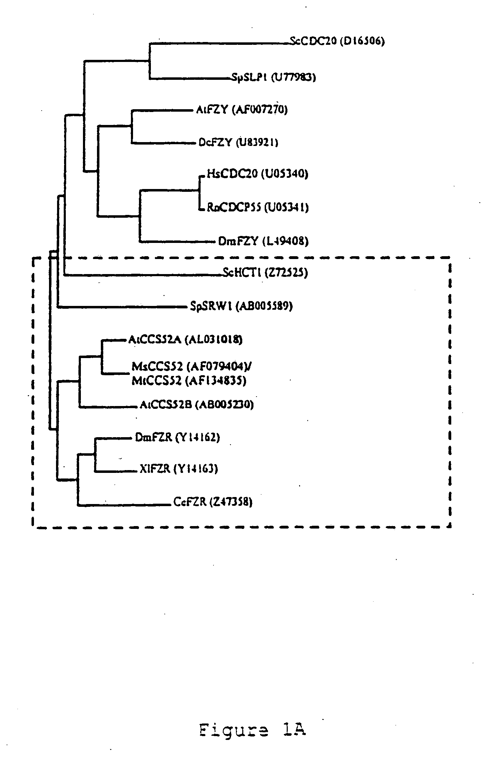

La figure 1A représente un dendrogramme de la famille des protéines à motifs WD40 répétés, qui montrent que les protéines CCS52 forment avec les autres protéines FZR, une sous-famille représentant une branche qui a évolué séparément de celles respectivement constituées par les protéines CDC20, P55, et fizzy.

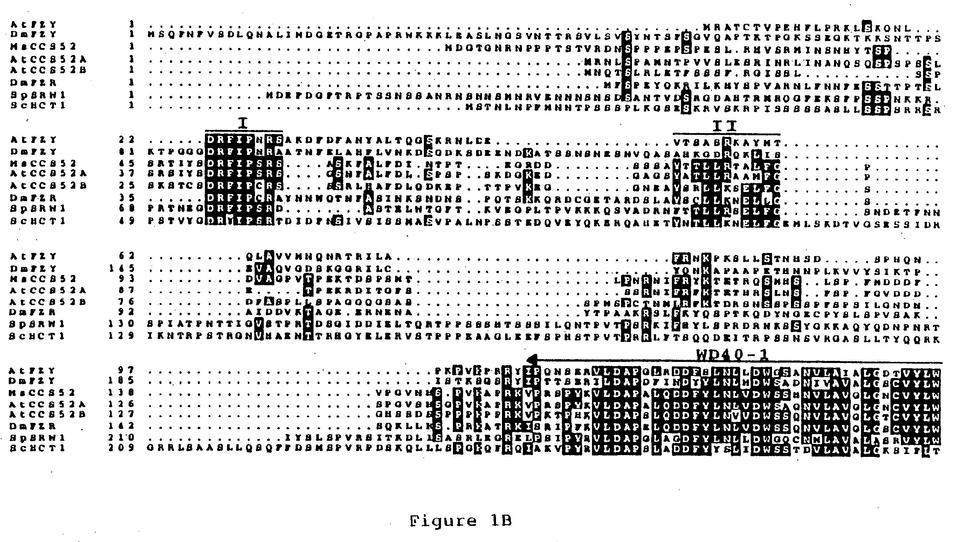

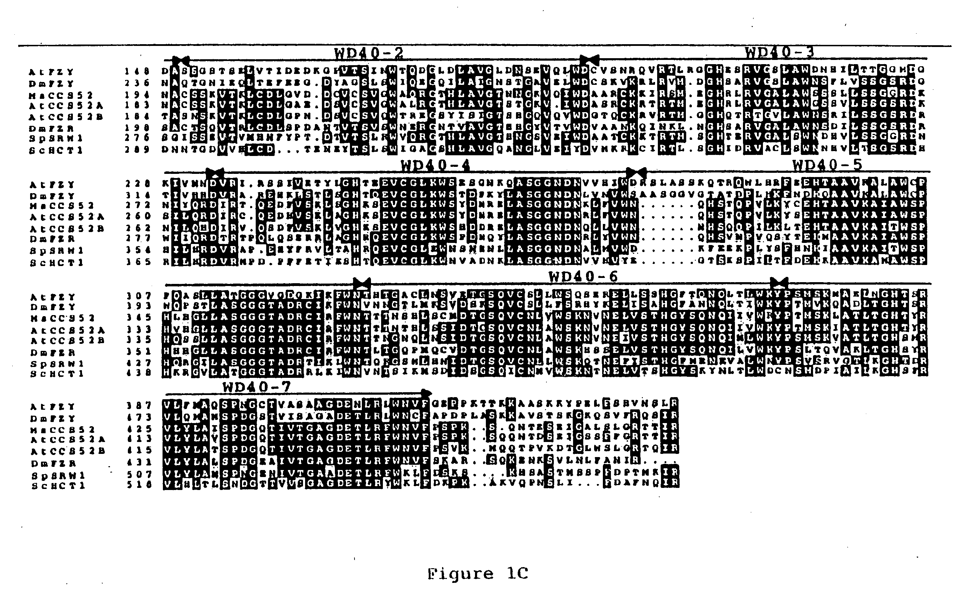

Les figures 1B et 1C représentent l'alignement, effectué en utilisant le logiciel « PRETTYBOX », de la séquence CCS52 de *Medicago sativa,* (MsCCS52) et des séquences FZY et FZR de Drosophile (DmFZY et DmFZR), HCT1 de *Saccharomyces cerevisiae* (ScHCT1), SRW1 de *Schizosaccharomyces pombe* (SpSRW1), FZY *d'Arabidopsis thaliana* (AtFZY), et des 2 polypeptides d'*Arabidopsis thaliana* (AtCCS52A = peptide déduit de AL031018, et AtCCS52B = peptide déduit de AB005230).

La protéine CCS52Ms contient 7 domaines à motifs WD40 répétés, situés dans les portions centrale et C-terminale de la molécule (l'emplacement de ces domaines numérotés de I à VII, est indiqué sur les figures 1B et 1C, au dessus de l'alignement des séquences). Ces domaines ne présentent que peu d'homologie entre eux, d'où l'on peut conclure qu'ils représentent des sites d'interaction avec des protéines différentes. Le dernier domaine (VII) comprend un site potentiel de liaison pour les cyclines.

Dans la partie N-terminale de la protéine CCS52Ms, sont localisées une séquence peptidique (DRFIPSR) qui correspond à un motif présent chez les protéines FZR ainsi que chez d'autres protéines à motifs WD40 répétés telles que cdc20, p55 et fizzy, ainsi qu'une séquence peptidique (AYTTLLRTALFG) qui correspond à un motif spécifique de la famille FZR, absent des autres protéines à motifs WD40 répétés (l'emplacement de ces motifs, respectivement dénommés I et II, est indiqué sur la figure 1B au-dessus de l'alignement des séquences).

Des sites potentiels de phosphorylation par des CDK (cyclin dependent kinases ou kinases cyclines-dépendantes), sont localisés dans la portion N-terminale, aux positions 43 (SPSR), 99 (TPEK), 144 (SPVK), 154 (RSP), et 155 (SPYK), ainsi que dans la portion C-terminale à la position 454 (SPK), de CCS52Ms. Les sites situés aux positions 43 et 144 sont également présents chez d'autres protéines FZR, tandis que les sites situés aux positions 99, 154, et 155 paraissent plus spécifique des protéines CCS52 de plantes ; le site C-terminal en position 454 apparaît également spécifique des protéines CCS52 de plantes.

Une séquence de 15 acides aminés RDNSPPPEPSPESLR commençant au résidu 16, et correspondant à un motif de dégradation protéique PEST est également présente dans la portion N-terminale de CCS52Ms. Ce motif permet probablement, par l'intermédiaire de la dégradation de CCS52, de réguler ses interactions avec d'autres protéines.

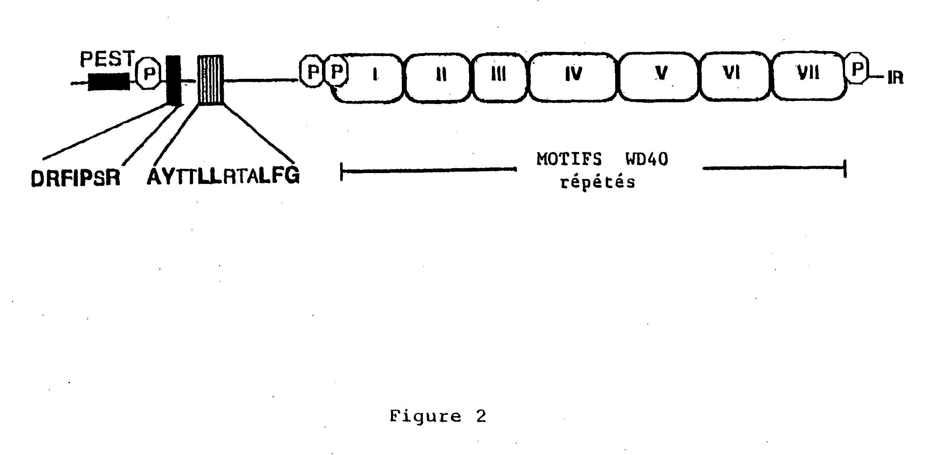

La structure de la protéine CCS52Ms est schématisée sur la figure 2, sur laquelle sont indiqués la position des motifs WD-40, des sites de phosphorylation (P), du motif PEST, et des motifs I et II.

La séquence de l'ADNc de *Medicago sativa* cloné par les inventeurs est représentée dans la liste de séquences en annexe sous le numéro SEQ ID NO:1; la séquence de la protéine CCS52Ms correspondante est représentée sous le numéro SEQ ID NO:2.

La région 3' non-traduite du transcrit de cet ADN comprend 2 séquences AUUUA, qui correspondent à des séquences d'instabilité des ARNm, et peuvent donc jouer un rôle pour réguler la quantité de transcrits de *ccs52.*

Les Inventeurs ont recherché la présence d'homologues de *ccs52Ms* par transfert de Southern, chez des espèces diploïdes et tétraploïdes de *Medicago,* ainsi que chez d'autres plantes, en particulier le tabac, la tomate, la pomme de terre, le soja, le blé et le riz : dans tous les cas plusieurs bandes ont été détectées, ce qui indique que *ccs52* représente bien une famille de gènes végétaux apparentée à la famille *fzr*.

Les Inventeurs ont étudié *in vivo* l'activité de la protéine CCS52Ms et ont montré qu'elle intervenait dans la régulation de la différenciation cellulaire, en favorisant l'endoréplication. En particulier, l'expression de la protéine CCS52Ms dans des plantes transgéniques induit chez celles-ci une augmentation de l'endoréplication et du niveau de ploïdie des cellules des plantes. Cet effet serait la conséquence d'un blocage de la mitose par l'activation de la dégradation des cyclines mitotiques, ce qui entraînerait une conversion des cycles mitotiques en endocycles constitués des phases G1-S-G2. La répétition des endocycles a pour résultat l'amplification du génome et l'augmentation de la ploïdie, corrélée à une augmentation du volume cellulaire.

La présente invention a pour objet une protéine végétale à motifs WD40 répétés, dénommée ci-après CCS52, favorisant l'endoréplication en induisant la dégradation des cyclines mitotiques, et présentant au moins 55% d'identité avec le polypeptide de séquence SEQ ID NO :2, caractérisée en ce qu'elle comprend les motifs peptidiques suivants : TPEK, RSP, SPYK, et SPK.

La présente invention englobe en particulier la protéine CCS52Ms définie par la séquence SEQ ID NO:2, la protéine CCS52Mt, dont la séquence diffère de celle de la protéine CCS52Ms par une substitution R/G en position 16 et une substitution V/I en position 141, et la protéine AtCCS52A dont la séquence, déduite du clone génomique AL031018 *d'Arabidopsis thaliana*, est représentée sur les Figures 1B et 1C.

La présente invention a également pour objet un vecteur recombinant, caractérisé en ce qu'il comprend une séquence codant pour une protéine CCS52, telle que définie ci-dessus, ou sa séquence complémentaire. Dans ce cadre, la présente invention englobe en particulier les ADNc et les ADN génomiques des protéines CCS52 telles que définies ci-dessus.

Des fragments d'acide nucléique codant pour une protéine CCS52, ou leurs complémentaires, peuvent être aisément identifiés et clonés en criblant des banques d'ADNc ou d'ADN génomique de plantes à l'aide d'oligonucléotides dérivés de la séquence de CCS52Ms, et notamment d'oligonucléotides dérivés des régions de cette séquence spécifique des protéines FZR, et en particulier des protéines CCS52.

Les protéines CCS52 peuvent être produites, en particulier, en exprimant ces séquences d'acide nucléique dans des cellules hôtes. La présente invention a également pour objet l'utilisation d'une protéine CCS52, telle que définie ci-dessus ou d'une séquence d'acide nucléique codant pour ladite protéine, ou de sa séquence complémentaire, pour réguler la différenciation et la prolifération de cellules végétales.

La modification de l'expression et/ou de l'activité de protéines CCS52 dans des cellules de plantes permet de modifier le cycle cellulaire, en favorisant soit la prolifération soit la différenciation, et de contrôler ainsi le processus de développement, afin d'obtenir par exemple une stimulation de l'embryogénèse somatique, d'augmenter la régénération in vitro de plantes à partir des cals, en augmentant la conversion en embryons, ou de favoriser le développement de certains organes, par exemple d'augmenter la productivité des tissus de réserve en augmentant leur endoploïdie.

On peut en particulier utiliser les séquences d'ADNc de protéines CCS52 ou des portions de ces séquences d'ADNc, ou de leur transcrits sens ou antisens ; il peut s'agir par exemple de la totalité d'une séquence codant pour une protéine CCS52Ms, ou d'une portion de cette séquence codante, et/ou de tout ou partie des régions 5' et 3' non traduites. Ces séquences peuvent être utilisées en orientation sens, ou si l'on souhaite inhiber l'expression de la protéine CCS52Ms dans une plante ou dans un tissu ou organe de celle-ci, en orientation antisens.

Généralement, ladite séquence d'acide nucléique sera placée sous contrôle transcriptionnel d'un promoteur approprié.

Avantageusement, on pourra ainsi utiliser un promoteur fort, pour augmenter, dans les cellules hôte, les niveaux d'expression de la protéine CCS52 ; il pourra s'agir d'un promoteur inductible ou bien d'un promoteur constitutif, d'un promoteur ubiquitaire, ou d'un promoteur tissu-spécifique.

L'utilisation de promoteurs inductibles permet d'obtenir un blocage de la mitose, et l'induction de l'endoréplication au moment souhaité. L'utilisation de promoteurs tissu-spécifiques permet de cibler l'action de la protéine CCS52 sur certains tissus et organes (par exemple, des tissus de réserve).

A titre d'exemples de promoteurs forts utilisables dans le cadre de la présente invention, on citera : le promoteur CaMV35S [BENFLY et al, Science, 250, pp. 959-966, (1990)] ; le promoteur 35S ; les promoteurs *Agrobacterium tumefaciens* T-DNA : nopaline synthase, octopine synthase, mannopine synthase, 1', 2' [SANDERS et al., Nucleic Acid Res., 15, pp. 1543-1558, (1987) ; HOOYKAAS and SCHILPEROORT, Plant. Mol. Biol., 19, pp. 15-38, (1992)].

A titre d'exemples de promoteurs inductibles utilisables dans le cadre de la présente invention, on citera : le promoteur inductible par la tétracycline [WEINMANN et al., Plant J., 5, pp. 559-569, (1994)] ; le promoteur inductible par le cuivre [METT et al., Transgenic Res., 5, pp. 105-113, (1996)] ; le promoteur inductible par les glucocorticoïdes [AOYAMA et CHUA, Plant. J., 11, pp. 605-612, (1997)].

A titre d'exemples de promoteurs tissu-spécifiques utilisables dans le cadre de la présente invention, on citera : le promoteur endosperme-spécifique [OPSAHL-FERSTAD et al., Plant J., 12, pp. 235-246, (1997) ; DOAN et al., Plant Mol. Biol., 31, pp. 877-886, (1996) ; les promoteurs nodosités-spécifiques *(enod12A*/*B* ou leghémoglobine) [TRINH et al., Plant Cell Reports, (17, pp. 345-355, (1998) ; VIJN et al., Plant Mol. Biol., 28, pp. 1103-1110, (1995)] ou bien des promoteurs précoces inductibles par le facteur Nod et des promoteurs tardifs (promoteur de la cycline D ou des nodulines tardives (type leghémoglobine) et promoteurs régulés par des hormones, tels que parA/B [TAKAHASHI et al., Proc. Natl. Acad. Sci. USA, 87, pp. 8013-8016, (1990)], *GH3* [LIU et al., Plant Cell, 6, pp. 645-657, (1994)].

L'Invention a également pour objet des cellules et des organismes pluricellulaires transformés par au moins une séquence d'acide nucléique codant pour une protéine CCS52, ou son complémentaire ; il s'agit en particulier de cellules végétales ou de végétaux.

La présente invention sera mieux comprise à l'aide du complément de description qui va suivre, et se réfère à des exemples non limitatifs illustrant l'identification, le clonage et l'expression du gène CCS52Ms.

### EXEMPLE 1 : CLONAGE ET SEQUENCAGE D'UN ADNC de CCS52Ms.

Un clone d'ADNC de CCS52Ms a été obtenu par criblage différentiel à partir d'une banque d'ADNC de nodosités de *Medicago sativa*, fortement stimulées pendant l'organogénèse nodulaire.

Le protocole suivant a été utilisé :

L'ADNc de *ccs52Ms* de *M. sativa* est isolé par la technique de DD-RT-PCR (Differential Display RT-PCR) [LIANG et PARDEE, Science, 257, pp. 967-971, (1992)], en utilisant les kits RNAimage^{®} (GENHUNTER CORPORATION). Les échantillons d'ARN sont isolés à partir de la zone racinaire sensible au facteur Nod de jeunes plants de *M. sativa* (croissance dans un milieu limité en nitrate), en l'absence de bactéries ou inoculés par des souches de *R. meliloti* Nod+ (EK1433) ou Nod- (EK133) pendant 4 jours. Le fragment de DD-RT-PCR *ccs52Ms*, présentant une augmentation de l'expression des nodosités; est cloné dans le vecteur de clonage pCT-TRAP (GENHUNTER CORPORATION) et utilisé comme sonde pour l'isolement des clones complets à partir d'une banque d'ADNc de nodosités de *M. sativa sp. varia* A2, construite dans λ-ZAP (STRATAGÈNE) (CRESPI et al., EMBO J., 1994, 13, 5099-5112).

Sept clones d'ADNc, obtenus à partir de 2.10⁵ phages représentent 2 types d'ADNc différant l'un de l'autre uniquement au niveau de 4 acides aminés (16R-G, 17D-N, 33S-N, 52R-G) et de la longueur du fragment 3'UTR. Une identité de 99% des clones, au niveau de la séquence en acides aminés, suggère qu'ils représentent des allèles du même gène chez *M. sativa* tétraploïde allogame.

Le séquençage des ADNc de *ccs52Ms* est effectué avec le système ABIprism de PERKIN-ELMER.

Les clones génomiques *ccs52Ms* et *ccs52Mt* sont isolés à partir de banques génomiques de *M. sativa* cv. Nagyszénasi et *M. trucatula* ecotype GHOR, en utilisant l'ADNc de *ccs52Ms* comme sonde d'hybridation. Ces banques génomiques sont construites par digestion partielle de l'ADN génomique avec l'enzyme de restriction MboI et le clonage des fragments d'ADN de taille comprise entre 15 et 20 Kb dans le site BamHI de λ-EMBL4.

### EXEMPLE 2 : IDENTIFICATION DE LA FAMILLE DU GENE CCS52MS DANS MEDICAGO ET SON EXPRESSION DANS DIFFERENTS ORGANES VEGETAUX.

L'existence de copies multiples du gène *ccs52* est recherchée par hybridation de type Southern dans des cultivars tétraploïdes de *M. sativa* Nagyszénasi et Cardinal et chez *M. truncatula* diploïde autogame, une plante modèle dans la recherche sur les légumes.

L'ADN des plantes est isolé à partir des feuilles jeunes, en utilisant le kit d'extraction NUCLEON PHYTOPURE DNA (AMERSHAM).

Les échantillons d'ADN sont digérés par EcoRI et transférés sur membrane de nylon BIOTRANS (+) (ICN).

L'hybridation de Southern est réalisée conformément aux protocoles classiques [(SAMBROOK, Molecular Cloning: A Laboratory Manual 2nd edn., Cold Spring Harbor Laboratory Press, New York, (1989) ; AUSUBEL, Current Protocols in Molecular Biology, (1989)], dans des conditions stringentes à 65°C (hybridation dans un tampon CG ; lavage : 2 x SSC, 0,1% SDS pendant 2 fois 15 mn, puis 0,5 x SSC, 0,1% SDS pendant 2 fois 30 min.).

L'expression de *ccs52Ms* est étudiée par analyse de Northern.

L'ARN total est isolé à partir de différents organes de *M. sativa* cultivar Sitel :
- à partir des racines, inoculées pendant 4 jours avec le mutant Nod- *R. meliloti* (EK133) et avec la souche surproductrice de facteurs Nod (EK1433) ;
- à partir des nodosités, 12, 19, 23 et 30 jours après une infection par *R. meliloti*, et,
- à partir des tiges, des hypocotyles, des feuilles, des bourgeons, des fleurs, des racines de plants de 3 jours, de 7 jours, des racines privées d'azote et ne présentant pas de pointes racinaires, des racines de 7 jours, sans pointes racinaires, mises en culture en présence de nitrate, des nodosités spontanée développées en l'absence de *R. meliloti,* et des pointes racinaires ou de culture de cellules de *M. sativa sp. varia* A2.

100 mg de chacun des organes testés, collectés sous azote liquide, sont utilisés pour l'extraction de l'ARN (RNEASY PLANT, QUIAGEN).

L'ARN est chargé (10 µg par ligne) sur un gel dénaturant (formaldéhyde) [SAMBROOK, Molecular Cloning: A Laboratory Manual 2nd edn., Cold Spring Harbor Laboratory Press, New York, (1989)].

Le transfert d'ADN est effectué dans une solution de transfert 10 x SSC [CHOMCZYNSKI et al., Analytical Biochemistry, 221, pp. 303-305, (1994)].

Aussi bien dans le cas de l'hybridation Southern que dans le cas de l'hybridation Northern, le fragment d'ADNc *ccs52Ms* est marqué avec du [α-³²P] dCTP (Kit MEGAPRIM, AMERSHAM). L'hybridation avec la sonde *Msc27* sert de contrôle pour le chargement de l'ARN [SAVOURE et al., EMBO J., 13, pp. 1093-1102, (1994)].

Les résultats du transfert de Southern montrent que la sonde s'hybride avec différents fragments EcoRI de l'ADN génomique de *M. sativa* ou *M. truncatula*, ce qui indique que *ccs52Ms* représente chez *Medicago*, une famille multigénique.

Les résultats du transfert de Northern obtenus avec l'ARN total de racines inoculées avec le mutant Nod-EK133 de *R. meliloti,* ou avec la souche EK1433 surproductrice de facteurs Nod et avec l'ARN extrait des nodosités, 12, 19, 23 et 30 jours après infection avec *R. meliloti* montrent qu'on n'observe dans l'ARN total de racines, qu'une faible quantité de transcrits, ce qui reflète la faible proportion des cellules impliquées dans l'organogénèse des nodosités par rapport au nombre total de cellules des racines. En revanche, dans les nodosités de différents âges on observe un niveau élevé de transcription, qui reflète la persistance des méristèmes apicaux et des zones de différenciation.

Les résultats de transfert de Northern obtenus avec les ARN totaux de : 1: culture de cellules de M. *sativa* sp. *varia* A2, 2: tiges, 3: hypocotyles, 4: feuilles, 5: bourgeons floraux, 6: fleurs, 7: racines de pousses de 3 jours, 8: racines de pousses de 7 jours, privées d'azote, dépourvues d'extrémité, 9: pointes racinaires de 7 jours, cultivées en présence de nitrates, dépourvues d'extrémité, 10: nodosités spontanées développées en absence de *R. melioti*, 11: nodosités fixatrices d'azote, 12: extrémités de pointes racinaires, montrent que l'expression du *ccs52Ms* n'est pas limitée aux nodosités, bien que cet organe soit celui qui contienne le niveau de transcrits le plus élevé.

Ces transcrits sont en effet présents en quantités variables pratiquement dans tous les organes, ce qui indique que cette protéine intervient dans le développement de chacun d'entre eux. Mis à part les nodosités, le niveau de transcription est également élevé chez les jeunes pousses, et dans les cultures cellulaires, où l'on détecte en outre un ARNm de plus petite taille qui peut correspondre soit à une polyadénylation différente, soit à l'expression d'une copie homologue du gène.

Des analyses par hybridation *in situ* ont également été effectuées, et montrent que l'ARNm de *ccs52Ms* est localisé principalement dans la zone de différenciation, et en particulier à l'interface entre les zones II et III de la nodosité, qui sont les régions où la différenciation est la plus active.

Parallèlement, on observe dans les mêmes zones une expression des cyclines de type G1 et mitotiques, ainsi que de l'histone H3 spécifique de la phase S.

Ceci indique que CCS52Ms intervient dans la régulation du cycle cellulaire, probablement d'une manière similaire à ses homologues de la levure et de la drosophile, c'est-à-dire par l'intermédiaire de la protéolyse de cyclines mitotiques, qui inhibe la mitose et induit des cycles d'endoréplication.

### EXEMPLE 3 : EXPRESSION DE CCS52MS CHEZ SCHIZOSACCHAROMYCES POMBE

L'expression de CCS52Ms a été étudiée chez *S. pombe* chez qui un homologue fonctionnel (SRW1) a été décrit récemment (YAMAGUCHI, publication précitée). Le gène codant pour CCS52Ms a été cloné dans le plasmide pREP1 sous contrôle du promoteur *nmt1* répressible par la thiamine.

L'ADNc de *ccs52Ms* obtenu après coupure de λ-ZAP (STRATAGÈNE) est digéré avec AgeI et partiellement avec EcoRV. Le fragment AgeI-EcoRV de 1,6 kb représentant la région codante, à l'exception des 4 premiers codons, est cloné dans un vecteur SKII BLUESCRIPT (STRATAGÈNE) digéré par XmaI (compatible avec AgeI) et EcoRV. A partir de ce plasmide (pSK52B), l'ADNc de *ccs52Ms* est coupé par digestion BamHI-EcoRV et cloné dans les sites BamHI-SmaI du plasmide pREP1 [MAUNDRELL et al., Gene, 123, pp. 127-30, (1993)]. Pour générer un cadre de lecture en phase avec le codon de traduction ATG présent dans le vecteur sous contrôle du promoteur *nmtI*, l'ADN est digéré avec BamHI et l'extrémité 5' est complétée en présence d'enzyme de Klenow et de dNTPs. La religature des extrémités à bouts francs entraîne une fusion correcte, également vérifiée par séquençage. Ce plasmide, dénommé pREP52, est utilisé pour transformer des cellules compétentes S. *pombe* SP-Q01 et les transformants sont sélectionnés sur des plaques d'agar EMM-thiamine, en utilisant le kit ESP (STRATAGÈNE). Les vecteurs pREP1 [MAUNDRELL et al., Gene, 123, pp. 127-30, (1993)] et pESP1 (STRATAGÈNE) sont utilisés comme contrôles négatifs ; le contrôle positif est constitué par *srw1* cloné dans pREP1 [YAMAGUSHI et al, Mol. Biol. Cell., 8, pp. 2475-2486, (1997)].

Les transformants de *S. pombe* SP-Q01 sont cultivés dans 2 ml de milieu EMM-thiamine 5 µM pendant 32 h à 30°C. Les cellules sont lavées 2 fois avec 10 ml d'eau stérile et remises en suspension dans 5 ml de milieu EMM. Les suspensions cellulaires sont divisées en deux moitiés : 2,5 ml sont cultivées avec de la thiamine et 2,5 ml sont cultivées sans thiamine, à 30°C. Des aliquots de cultures sont prélevés après 16 h et 24 h de culture et fixés avec de l'éthanol, colorés avec du DAPI ou de l'iodure de propidium pour une analyse en cytométrie de flux et en microscopie [BEACH et al, Curr. Genet., 10, pp. 297-311, (1985)].

En présence de thiamine, l'expression de CCS52Ms est réprimée et on observe une croissance normale.

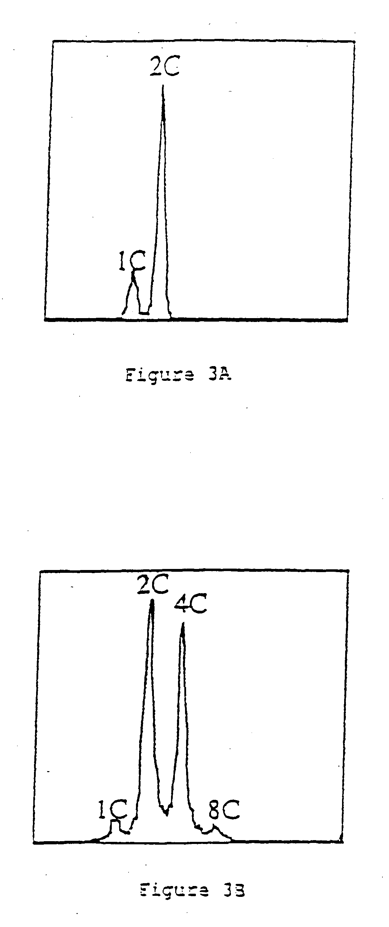

En l'absence de thiamine, l'expression de CCS52Ms entraîne l'inhibition de la croissance de *S. pombe*, qui s'accompagne d'une endoréplication comme l'illustre la figure 3B, qui montre la présence de noyaux ≥ 4C, qui n'est pas observée dans les cellules contrôles de *S. pombe*, portant le vecteur vide pREP1 (figure 3A).

La morphologie des cellules est également modifiée par l'expression de CCS52Ms. On observe un allongement des cellules et une augmentation de la taille des noyaux, identiques à ceux observés lors de l'expression de SRW1 [YAMAGUSHI et al, Mol. Biol. Cell., 8, pp. 2475-2486, (1997)], tandis qu'aucun changement morphologique n'est observé lorsque *S. pombe* ne porte que le vecteur pREP1.

Chez *S. pombe*, SRW1 est essentiel pour la dégradation de la cycline mitotique CDC13. Pour vérifier si CCS52 agit de la même manière, la quantité de CDC13 a été évaluée dans des cultures d'une souche (SY1) de *S. pombe*, porteuse d'une délétion dans le gène *srw1*, et ne dégradant pas CDC13.

Les protéines totales obtenues à partir de cultures de SY1 transformées avec pREP1 (témoin) ou avec pREP1-*ccs52* ont été analysées par transfert de Western, et révélation à l'aide d'anticorps anti-CDC13.

Parallèlement, l'expression de la kinase CDC2 et celle de l'α-tubuline ont respectivement été évaluées par révélation à l'aide d'anticorps anti-PSTAIR et anti α-tubuline.

Les résultats obtenus montrent une réduction très importante de la CDC13 dans les cellules transformées avec pREP1-*ccs52* par rapport aux cellules témoin. En revanche, il n'y a aucune variation de la CDC2 et de l'α-tubuline.

Ces résultats confirment que CCS52 est un équivalent fonctionnel de SRW1.

### EXEMPLE 4 : OBTENTION DE PLANTES TRANSGENIQUES TRANSFORMEES PAR LE GENE CCS52MS.

### 1° Expression d'un transcrit antisens et son action sur le niveau de ploïdie de Medicago truncatula.

Dans un premier temps, le niveau de ploïdie de différents organes de *Medicago truncatula* (plante naturellement diploïde) a été déterminé, par cytométrie de flux, chez des plantes non-transformées.

La technique utilisée est la suivante :
l'ADN nucléaire des plantes fraîchement récoltées est analysé par cytométrie de flux (EPICS V, Coulter), conformément à la méthode de BROWN et al., (A laboratory guide for Cellular and Molecular plant Biology, 1991, 326-345, ed. Negrutiu et al., Birkhäuser, Basel), modifiée de telle sorte que les noyaux soient colorés avec du DAPI à une concentration finale de 5 µg/ml. Le tampon nucléaire I est utilisé à 1% de Triton X-100 pour les nodosités.

Dans les jeunes pousses, on trouve une quantité d'ADN de 2C à 8C dans la racine et le cotylédon, alors que l'hypocotyle contient également des noyaux à 16C. Chez les plantes adultes, les feuilles sont diploïdes, contenant 95% de noyaux à 2C et 5% de noyaux à 4C. Dans les pétioles et les nodosités, des noyaux de 2C à 32C ont été détectés. Toutefois, le pétiole contient majoritairement des noyaux à 2C, alors que les nodosités contiennent majoritairement des noyaux à 4C.

Un fragment SstI-PvuII de 1,2kb contenant les 3/4 de la séquence codante de *ccs52Ms*, a été placé en orientation antisens sous contrôle du promoteur 35S, dans un vecteur binaire obtenu à partir du vecteur pGPTV-BAR, portant le gène *bar* de résistance à l'herbicide BASTA comme marqueur de sélection, et des sites de clonage multiple. Cette construction est obtenue en insérant le promoteur 35S dans un fragment HindIII-XbaI (obtenu à partir de pBI121, CLONTECH), dans les sites HindIII-XbaI du vecteur pGPTV-BAR. Le gène *uidA* est ensuite éliminé du plasmide pGPTV-BAR par digestion XbaI-SstI au niveau du site multiple de clonage.

Pour obtenir la construction antisens de *ccs52Ms*, le fragment SstI-PvuII de 1,2 kb est cloné au niveau des sites SmaI-SstI du vecteur binaire ainsi obtenu.

Ces plasmides ainsi qu'un plasmide contrôle, contenant le gène *gus* au lieu de la construction *ccs52* antisens ont été introduits dans *Agrobacterium tumefaciens* (EHA105) par électroporation et utilisés pour transformer *Medicago truncatula* R108-1 selon le protocole décrit par HOFFMANN et al. [Mol. Plant Microbe Interaction, 10, pp. 307-315, (1997)] ; TRINH ET AL. [Plant Cell Reports, 17, pp. 345-355, (1998)].

Le niveau de ploïdie des plantes transgéniques obtenues a été analysé, comme décrit ci-dessus et le niveau de transcrits endogènes a été évalué par RT-PCR. Pour discriminer les transcrits endogènes de *ccs52Mt* des transcrits antisens, on utilise pour les transcrits endogènes la paire d'amorces P55CL/P55CR et pour les transcrits antisens, la paire d'amorces PSSBL/P55CR.
P55BL : TTTGGGGGTTGATGATTGTG
P55CL : CTCTCTACCGTTCTATCTCTTGGGA
P55CR : GGTAAAGATGCTACTTTGGTGGTGT

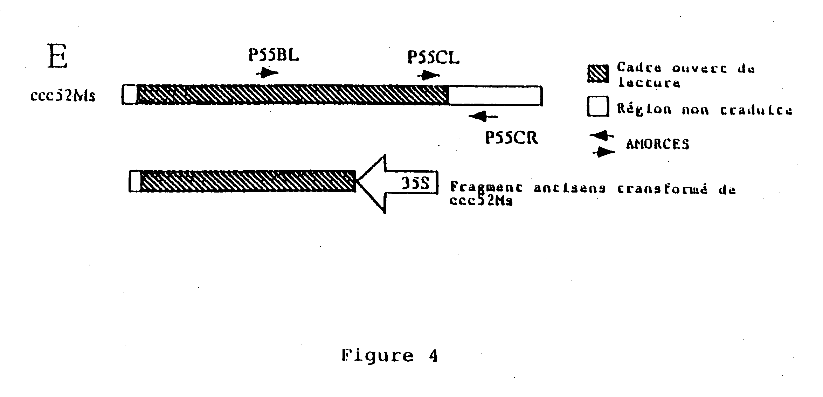

La position de ces amorces est schématisée sur la figure 4.

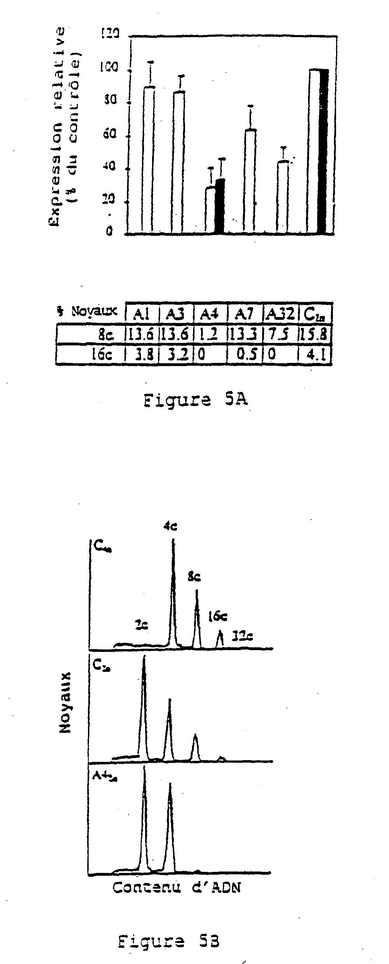

La figure 5A montre les résultats d'évaluation de la quantité de transcrits *ccs52Mt* endogènes :
- par RT-PCR (□) dans les lignées transgéniques A1 A3 A4 A7 et A32 et dans les plantes témoin contenant le gène gus (C₂ₙ), et
- par transfert de Northern (■) dans les plantes A4 et C₂ₙ.

Les résultats d'analyse en cytométrie de flux sont illustrés par la figure 5B, pour les pétioles de plantes témoin contenant le gène *gus*, diploïdes (C₂ₙ) ou tétraploïdes (C₄ₙ), et de plantes de la lignée A4.

Sur 38 plantes transgéniques régénérées, 3(A4, A7 et A32) ont montré une endoploïdie significativement réduite, et notamment la plante A4. C'est également dans cette lignée que le niveau d'expression des transcrits endogènes de *ccs52Ms* est le plus faible, comme le montre la figure 5B. Le fait qu'une réduction de l'endoploïdie n'ait jamais été observée auparavant chez d'autres plantes transgéniques et ne soit pas observée chez les plantes témoin, permet d'attribuer ce phénomène à l'altération de l'expression de CCS52Ms, et non à un effet secondaire de la transgénèse.

En outre, la plante A4 produit une quantité de graines significativement inférieure à celle des plantes témoin. D'autre part, elle forme moins de rameaux latéraux, et n'a développé que 2 nodules au niveau des racines, au lieu des 50 nodules en moyenne développés par les plantes témoin cultivées dans les mêmes conditions.

L'impact de la suppression partielle de l'expression de *ccs52* sur le développement des organes de la plante a également été déterminé. Dans ce but, la largeur des pétioles a été mesurée et corrélée avec de pourcentages de noyaux endorépliqués (>4C), chez la génération T1 issue de la lignée A4 et chez les plantes témoins C₂ₙ et C₄ₙ.

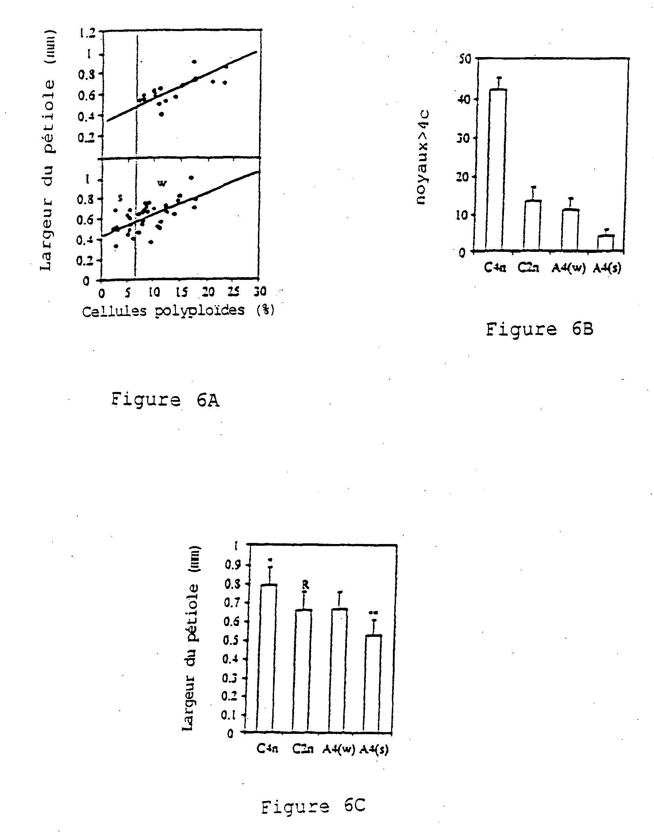

Les résultats sont illustrés par la Figure 6. La Figure 6A qui représente la largeur du pétiole en fonction du pourcentage de cellules polyploïdes montre que, chez les plantes témoin C₂ₙ (18 plantes), la largeur des pétioles varie en corrélation avec le nombre de cellules diploïdes. Dans les plantes issues de A4 (36 plantes), on observe à la fois une variation plus réduite de la taille des pétioles et un pourcentage plus faible de cellules polyploïdes, ce qui indique que le degré d'endoploïdie peut affecter directement la taille finale des organes végétaux.

12 des 36 plantes T1 issues de A4 contiennent moins de 6% de noyaux endorépliqués (>4C) dans leurs pétioles (Figure 6B). Ces plantes [A4(s)] ont été regroupées et analysées séparément du reste des plantes A4 T1 [A4(w)] qui présentent des altérations phénotypiques moins importantes.

La Figure 6C montre que la largeur des pétioles chez les plantes A4(w) est comparable à celle des plantes témoin diploïdes C₂ₙ ; en revanche, la largeur des pétioles chez les plantes A4(s) est significativement inférieure à celle des plantes témoin diploïdes C₂ₙ·et la largeur des pétioles chez les plantes témoin tétraploïdes C₄ₙ, est significativement supérieure à celle observée chez les plantes diploïdes.

La taille des feuilles (qui ne contiennent pas de cellules endorépliquées et dont l'endoploïdie n'est donc pas affectée par le niveau d'expression de CCS52) a également été mesurée. Dans ce cas on n'observe aucune différence significative entre les plantes A4(w), A4(s), et les plantes témoin diploïdes C₂ₙ. en revanche la taille des feuilles est significativement plus importante chez les plantes témoin tétraploïdes C₄ₙ.

Ces résultats montrent que l'endoploïdie affecte la taille des organes végétaux, et que la modification de l'expression de CCS52 agit à ce niveau par l'intermédiaire d'une modification de l'endoploïdie.

### 2° Expression de la protéine CCS52Ms dans des plantes transgéniques.

Des vecteurs d'expression contenant le gène *ccs52Ms* sous contrôle du promoteur 35S, ainsi que des vecteurs d'expression comprenant le gène *ccs52Ms*, sous contrôle d'un promoteur tissu-spécifique, ont été construits selon le protocole suivant :

Pour l'expression tissu-spécifique de CCS52Ms, l'ADNc est placé sous le contrôle des promoteurs *enod12AMs* et *Srglb3* décrits par TRINH et al. [Plant Cell Reports, 17, pp. 345-355, (1998)], en utilisant comme vecteur pISV-BMCS, un dérivé de pISV2301, et, au lieu du promoteur *enod12AMs* complet, seulement un fragment de 0,3 kb de celui-ci, considéré comme suffisant pour une expression nodosité-spécifique [VIJN et al., Plant Mol. Biol., 28, pp. 1103-1110, (1995)].
Construction de pISV-BMCS : pISV2301 est digéré par HindIII et SstI pour éliminer la séquence du promoteur 2X35S-AMV, qui est remplacé par l'oligonucléotide BMCS double-brin suivant :
AGCTTCCCGGGGGAGCTCTAGACTCGAGCAGCT
AGGCCCCTCGAGATCTGAGCTCG.

Cet oligonucléotide contient les sites SmaI, SstI, XbaI, XhoI.

pISV-BMCS12A est construit par clonage dans pISV-BMCS d'un fragment du promoteur *enod12AMs* de 0,3 kb; obtenu à partir du plasmide pPR89 [BAUER et al., Plant J., 10, pp. 91-105, (1996)].

pISV-BMCS-LB3 est construit par digestion de pISV-BMCS avec HindIII-SstI et clonage d'un fragment HindIII-SstI contenant le promoteur leghémoglobine de *Sesbania rostrata* à partir de pLP32 [TRINH et al, Plant Cell Reports, 17, pp. 345-355, (1998)].

Ces vecteurs ont été utilisés pour transformer *Medicago truncatulata* selon le protocole décrit ci-dessus pour les séquences antisens.

Lors de la régénération des plantes transgéniques, on observe une conversion des cals en embryons significativement plus importante chez les plantes transformées avec les constructions exprimant le gène *ccs52Ms*, que chez les plantes transformées avec la construction témoin, ce qui indique un effet positif de CCS52Ms sur l'embryogénèse somatique.

## Revendications

1. Protéine à motifs WD40 répétés favorisant l'endoréplication en induisant la dégradation des cyclines mitotiques, et présentant au moins 55% d'identité avec le polypeptide de séquence SEQ ID NO :2, **caractérisée en ce qu'**elle comprend les motifs peptidiques suivants : TPEK, RSPYK, et SPK.

2. Protéine selon la revendication 1, **caractérisée en ce qu'**elle est choisie parmi : la protéine MsCCS52 définie par la séquence SEQ ID NO : 2 ; la protéine AtCCS52A dont la séquence est représentée sur les Figures 1 B et 1 C.

3. Vecteur recombinant contenant un polynucléotide choisi parmi :
a) un polynucléotide codant pour une protéine selon une quelconque des revendications 1 ou 2 ;
b) un polynucléotide complémentaire du polynucléotide a).

4. Cellule transformée par au moins un polynucléotide tel que défini dans la revendication 3.

5. Cellule transformée selon la revendication 4, **caractérisée en ce qu'**il s'agit d'une cellule végétale.

6. Plante transgénique transformée par au moins un polynucléotide tel que défini dans la revendication 3.

7. Utilisation d'une protéine selon l'une quelconque des revendications 1 ou 2, ou d'un polynucléotide tel que défini dans la revendication 3, pour réguler la différenciation et la prolifération de cellules végétales.

8. Utilisation selon la revendication 7, **caractérisée en ce que** ladite protéine ou ledit polynucléotide est utilisé(e) pour favoriser l'endoploïdie dans les cellules d'une plante ou d'un tissu végétal.

9. Utilisation selon la revendication 7, **caractérisée en ce que** ladite protéine ou ledit polynucléotide est utilisé(e) pour favoriser la régénération *in vitro* de plantes à partir de cals en culture.

## Claims

1. Protein having repeat WD40 motifs promoting endoreplication by inducing degradation of mitotic cyclins and being at least 55% identical to the polypeptide sequence SEQ ID NO: 2, **characterised in that** it contains the following peptide motifs: TPEK, RSPYK and SPK.

2. Protein according to claim 1, **characterised in that** it is chosen from: protein MsCCS52 defined by the sequence SEQ ID NO: 2; protein AtCCS52A whose sequence is shown in Figures 1B and 1C.

3. Recombinant vector containing a polynucleotide chosen from:
a) a polynucleotide coding for a protein according to one of claims 1 or 2;
b) a complementary polynucleotide of polynucleotide a).

4. Cell transformed by at least one polynucleotide as defined in claim 3.

5. Transformed cell according to claim 4, **characterised in that** it is a plant cell.

6. Transgenic plant transformed by at least one polynucleotide as defined in claim 3.

7. Use of a protein according to one of claims 1 or 2 or of a polynucleotide as defined in claim 3 for regulating the differentiation and the proliferation of plant cells.

8. Use according to claim 7, **characterised in that** said protein or said polynucleotide is used to promote endoploidy in the cells of a plant or a plant tissue.

9. Use according to claim 7, **characterised in that** said protein or said polynucleotide is used to promote *in vitro* regeneration of plants from callus culture.

## Patentansprüche

1. Protein mit wiederholten WD40-Motiven, das die Endoreplikation durch Induktion des Abbaus der mitotischen Cycline begünstigt und mindestens 55% Identität mit dem Polypeptid der Sequenz SEQ ID NO: 2 besitzt, **dadurch gekennzeichnet, dass** es die folgenden Peptidmotive umfasst: TPEK, RSPYK und SPK.

2. Protein nach Anspruch 1, **dadurch gekennzeichnet, dass** es aus dem Protein MsCCS52, das durch die Sequenz SEQ ID NO: 2 definiert ist, dem Protein AtCCS52A, dessen Sequenz in den Figuren 1B und 1C dargestellt ist, ausgewählt ist.

3. Rekombinanter Vektor, enthaltend ein Polynucleotid, ausgewählt aus:
a) einem Polynucleotid, das ein Protein nach einem der Ansprüche 1 oder 2 codiert,
b) einem Polynucleotid, das zu dem Polynucleotid a) komplementär ist.

4. Durch mindestens ein Polynucleotid nach Anspruch 3 transformierte Zelle.

5. Transformierte Zelle nach Anspruch 4, **dadurch gekennzeichnet, dass** es sich um eine Pflanzenzelle handelt.

6. Transgene Pflanze, die durch mindestens ein Polynucleotid nach Anspruch 3 transformiert ist.

7. Verwendung eines Proteins nach einem der Ansprüche 1 oder 2 oder eines Polynucleotids nach Anspruch 3 zur Regulierung der Differenzierung und der Proliferation von Pflanzenzellen.

8. Verwendung nach Anspruch 7, **dadurch gekennzeichnet, dass** das Protein oder das Polynucleotid verwendet wird, um die Endoploidie in den Zellen einer Pflanze oder eines Pflanzengewebes zu begünstigen.

9. Verwendung nach Anspruch 7, **dadurch gekennzeichnet, dass** das Protein oder das Polynucleotid verwendet wird, um die *in vitro*-Regeneration von Pflanzen aus Callus-Kulturen zu begünstigen.


### LISTE DE SEQUENCES

<110> CNRS
   KONDOROSI, Eva
   CEBOLLA, Angel
   KONDOROSI, Adam
<120> Protéine végétale à motifs WD40 répétés, acide nucléique codant pour ladite protéine, et leurs applications.
<130> MJPcb644/39
<140>
   <141>
<150> FR9807174
   <151> 1998-06-08
<160> 2
<170> PatentIn Ver. 2.1
<210> 1
   <211> 2006
   <212> ADN
   <213> Medicago sativa
<220>
   <221> CDS
   <222> (182)..(1609)
<400> 1
   gattcggcac gaggaagaaa caaagaaact ctctctctct atttctttct ctctgcacaa 60
   ttttcgagta gtgttatttt ttaataaaaa attaattaat ttttttttat ataaaagccg 120
   tgcaaaaaat tcttttacag cgttcttttt tccccgggaa aaaaattaac acagctccgc 180
   c atg gac gga acc ggt aat cga aat cca cca ccg act tcc acc gtc aga 229
     Met Asp Gly Thr Gly Asn Arg Asn Pro Pro Pro Thr Ser Thr Val Arg
       1               5                  10                  15
   gac aat tct cca ccg cct gag cca tca ccg gag agt ctc cgt cat gta    277
   Asp Asn Ser Pro Pro Pro Glu Pro Ser Pro Glu Ser Leu Arg His Val
               20                  25                  30
   agc cgt atg atc aac agc aac cat tac acc tca cct tct cga aca atc    325
   Ser Arg Met Ile Asn Ser Asn His Tyr Thr Ser Pro Ser Arg Thr Ile
           35                  40                  45
   tac tcc gat agg ttc att ccg agt aga tct gct tcg aaa ttc gct ttg    373
   Tyr Ser Asp Arg Phe Ile Pro Ser Arg Ser Ala Ser Lys Phe Ala Leu
       50                  55                  60
   ttt gat atc aat act ccg aca gaa gga cgc gat gat agt tcc agc gct    421
   Phe Asp Ile Asn Thr Pro Thr Glu Gly Arg Asp Asp Ser Ser Ser Ala
   65                  70                  75                  80
   tat acg act ctt ctg aga acg gcg ttg ttt gga ccg gat gtt gcc ggt    469
   Tyr Thr Thr Leu Leu Arg Thr Ala Leu Phe Gly Pro Asp Val Ala Gly
                   85                  90                  95
   ccg gtt acg ccg gaa aaa acc gac tcg ccg tcg atg aca ttg ccg aat    517
   Pro Val Thr Pro Glu Lys Thr Asp Ser Pro Ser Met Thr Leu Pro Asn
               100                 105                 110
   agg aat att ttt agg tat aag acg gag acg aga cag tcc atg cac tcg    565
   Arg Asn Ile Phe Arg Tyr Lys Thr Glu Thr Arg Gln Ser Met His Ser
           115                 120                 125
   ctt tcg ccg ttt atg gat gat gat ttt gtt cct ggt gtt aat cat agt    613
   Leu Ser Pro Phe Met Asp Asp Asp Phe Val Pro Gly Val Asn His Ser
       130                 135                 140
   ccg gtt aag gct cct agg aag gtt cct cga tcg cct tat aag gtt ttg    661
   Pro Val Lys Ala Pro Arg Lys Val Pro Arg Ser Pro Tyr Lys Val Leu
   145                 150                 155                 160
   gat gca cct gct ttg caa gat gat ttt tat ctg aat ctg gta gat tgg    709
   Asp Ala Pro Ala Leu Gln Asp Asp Phe Tyr Leu Asn Leu Val Asp Trp
                   165                 170                 175
   tct tca cac aat gtg ttg gct gtt ggt ttg ggt aac tgt gtc tat ctc    757
   Ser Ser His Asn Val Leu Ala Val Gly Leu Gly Asn Cys Val Tyr Leu
               180                 185                 190
   tgg aat gct tgt agc agc aag gta act aaa tta tgt gat ttg ggg gtt    805
   Trp Asn Ala Cys Ser Ser Lys Val Thr Lys Leu Cys Asp Leu Gly Val
           195                 200                 205
   gat gat tgt gtt tgt tct gtt ggt tgg gct caa cgt ggt act cat ctt    853
   Asp Asp Cys Val Cys Ser Val Gly Trp Ala Gln Arg Gly Thr His Leu
       210                 215                 220
   gct gtt gga act aac aat ggt aaa gtt cag att tgg gat gca gca aga    901
   Ala Val Gly Thr Asn Asn Gly Lys Val Gln Ile Trp Asp Ala Ala Arg
   225                 230                 235                 240
   tgc aag aag ata aga tca atg gag ggc cat cgg tta cgt gtc ggg gcc    949
   Cys Lys Lys Ile Arg Ser Met Glu Gly His Arg Leu Arg Val Gly Ala
                   245                 250                 255
   ttg gcc tgg agt tca tct ctt ttg tct tct ggt gga cgg gat aag aat    997
   Leu Ala Trp Ser Ser Ser Leu Leu Ser Ser Gly Gly Arg Asp Lys Asn
               260                 265                 270
   att tat caa cga gat ata cgc aca caa gaa gat ttt gtt agt aaa ctg    1045
   Ile Tyr Gln Arg Asp Ile Arg Thr Gln Glu Asp Phe Val Ser Lys Leu
           275                 280                 285
   tca gga cac aaa tca gag gtt tgt gga ctg aag tgg tca tat gat aac    1093
   Ser Gly His Lys Ser Glu Val Cys Gly Leu Lys Trp Ser Tyr Asp Asn
       290                 295                 300
   cgt gag ttg gca tct gga gga aat gac aac aaa ttg ttt gtt tgg aat    1141
   Arg Glu Leu Ala Ser Gly Gly Asn Asp Asn Lys Leu Phe Val Trp Asn
   305                 310                 315                 320
   caa cac tca acc cag cct gtc ctc aag tac tgt gag cac aca gca gct 1189
   Gln His Ser Thr Gln Pro Val Leu Lys Tyr Cys Glu His Thr Ala Ala
   325 330 335
   gtt aaa gct att gca tgg tct cct cat ctt cat gga ctt ctt gca tct 1237
   Val Lys Ala Ile Ala Trp Ser Pro His Leu His Gly Leu Leu Ala Ser
   340 345 350
   gga gga gga act gca gat aga tgt att cgt ttt tgg aat aca acc aca 1285
   Gly Gly Gly Thr Ala Asp Arg Cys Ile Arg Phe Trp Asn Thr Thr Thr
   355 360 365
   aac tca cac ctt agc tgt atg gac act gga agt cag gtt tgc aat ctt 1333
   Asn Ser His Leu Ser Cys Met Asp Thr Gly Ser Gln Val Cys Asn Leu
   370 375 380
   gtc tgg tcc aaa aat gtc aac gaa cta gta agc aca cat ggg tac tcc 1381
   Val Trp Ser Lys Asn Val Asn Glu Leu Val Ser Thr His Gly Tyr Ser
   385 390 395 400
   cag aac cag att att gtt tgg aga tac ccc act atg tca aag ctg gcg 1429
   Gln Asn Gln Ile Ile Val Trp Arg Tyr Pro Thr Met Ser Lys Leu Ala
   405 410 415
   act ctt acc ggc cat act tat agg gtt ctc tat ctt gcc atc tct cca 1477
   Thr Leu Thr Gly His Thr Tyr Arg Val Leu Tyr Leu Ala Ile Ser Pro
   420 425 430
   gat gga cag act att gta act gga gct gga gat gaa acg ctt agg ttc 1525
   Asp Gly Gln Thr Ile Val Thr Gly Ala Gly Asp Glu Thr Leu Arg Phe
   435 440 445
   tgg aat gtt ttc cct tcc cct aaa tca cag aat act gaa agt gaa atc 1573
   Trp Asn Val Phe Pro Ser Pro Lys Ser Gln Asn Thr Glu Ser Glu Ile
   450 455 460
   gga gca tta tct ctt gga aga act act atc agg tga ttgatcctgg 1619
   Gly Ala Leu Ser Leu Gly Arg Thr Thr Ile Arg
   465 470 475
   cgttgcagcc caatcatgtg gcatatttct aagtttgggt tgctgtgtag aactaaattt 1679
   ctgagcggag aacaccatgg tggaaaaacc ttgaatataa aaacaccacc aaagtagcat 1739
   ctttaccaac tgggagagcc ttggagggag ctataaaagt tttgatatgg ctgccggtga 1799
   tattcctgca ttcatgtgta gtctcatttt atattgaaaa gatgataaca aatgggtaat 1859
   ttattgtctt ggacttatac atgcattgat ggagttgtag ccaagttttt ttattactct 1919
   ttttttcttt cttctttttg atagtgctct cctgcattat ttatataatt ttaagatgcg 1979
   ttaacagaga aaaaaaaaaa aaaaaaa 2006
<210> 2
   <211> 475
   <212> PRT
   <213> Medicago sativa
<400> 2
   Met Asp Gly Thr Gly Asn Arg Asn Pro Pro Pro Thr Ser Thr Val Arg
   1 5 10 15
   Asp Asn Ser Pro Pro Pro Glu Pro Ser Pro Glu Ser Leu Arg His Val
   20 25 30
   Ser Arg Met Ile Asn Ser Asn His Tyr Thr Ser Pro Ser Arg Thr Ile
   35 40 45
   Tyr Ser Asp Arg Phe Ile Pro Ser Arg Ser Ala Ser Lys Phe Ala Leu
   50 55 60
   Phe Asp Ile Asn Thr Pro Thr Glu Gly Arg Asp Asp Ser Ser Ser Ala
   65 70 75 80
   Tyr Thr Thr Leu Leu Arg Thr Ala Leu Phe Gly Pro Asp Val Ala Gly
   85 90 95
   Pro Val Thr Pro Glu Lys Thr Asp Ser Pro Ser Met Thr Leu Pro Asn
   100 105 110
   Arg Asn Ile Phe Arg Tyr Lys Thr Glu Thr Arg Gln Ser Met His Ser
   115 120 125
   Leu Ser Pro Phe Met Asp Asp Asp Phe Val Pro Gly Val Asn His Ser
   130 135 140
   Pro Val Lys Ala Pro Arg Lys Val Pro Arg Ser Pro Tyr Lys Val Leu
   145 150 155 160
   Asp Ala Pro Ala Leu Gln Asp Asp Phe Tyr Leu Asn Leu Val Asp Trp
   165 170 175
   Ser Ser His Asn Val Leu Ala Val Gly Leu Gly Asn Cys Val Tyr Leu
   180 185 190
   Trp Asn Ala Cys Ser Ser Lys Val Thr Lys Leu Cys Asp Leu Gly Val
   195 200 205
   Asp Asp Cys Val Cys Ser Val Gly Trp Ala Gln Arg Gly Thr His Leu
   210 215 220
   Ala Val Gly Thr Asn Asn Gly Lys Val Gln Ile Trp Asp Ala Ala Arg
   225 230 235 240
   Cys Lys Lys Ile Arg Ser Met Glu Gly His Arg Leu Arg Val Gly Ala
   245 250 255
   Leu Ala Trp Ser Ser Ser Leu Leu Ser Ser Gly Gly Arg Asp Lys Asn
   260 265 270
   Ile Tyr Gln Arg Asp Ile Arg Thr Gln Glu Asp Phe Val Ser Lys Leu
   275 280 285
   Ser Gly His Lys Ser Glu Val Cys Gly Leu Lys Trp Ser Tyr Asp Asn
   290 295 300
   Arg Glu Leu Ala Ser Gly Gly Asn Asp Asn Lys Leu Phe Val Trp Asn
   305 310 315 320
   Gln His Ser Thr Gln Pro Val Leu Lys Tyr Cys Glu His Thr Ala Ala
                   325                 330                 335
   Val Lys Ala Ile Ala Trp Ser Pro His Leu His Gly Leu Leu Ala Ser
               340                 345                 350
   Gly Gly Gly Thr Ala Asp Arg Cys Ile Arg Phe Trp Asn Thr Thr Thr
           355                 360                 365
   Asn Ser His Leu Ser Cys Met Asp Thr Gly Ser Gln Val Cys Asn Leu
       370                 375                 380
   Val Trp Ser Lys Asn Val Asn Glu Leu Val Ser Thr His Gly Tyr Ser
   385                 390                 395                 400
   Gln Asn Gln Ile Ile Val Trp Arg Tyr Pro Thr Met Ser Lys Leu Ala
                   405                 410                 415
   Thr Leu Thr Gly His Thr Tyr Arg Val Leu Tyr Leu Ala Ile Ser Pro
               420                 425                 430
   Asp Gly Gln Thr Ile Val Thr Gly Ala Gly Asp Glu Thr Leu Arg Phe
           435                 440                 445
   Trp Asn Val Phe Pro Ser Pro Lys Ser Gln Asn Thr Glu Ser Glu Ile
       450                 455                 460
   Gly Ala Leu Ser Leu Gly Arg Thr Thr Ile Arg
   465                 470                 475